# EUROPEAN PATENT SPECIFICATION

(11) **EP 1 748 734 B1**
(45) Date of publication and mention of the grant of the patent: **16.01.2008**
(21) Application number: 05730306.7
(22) Date of filing: 29.03.2005
(51) Int. Cl.: A61B 17/04

(54) **SURGICAL INSTRUMENT**
OPERATIONSINSTRUMENT
INSTRUMENT CHIRUGICAL

(30) Priority: 31.03.2004 NL 1025852; 29.12.2004 US 25727
(43) Date of publication of application: 07.02.2007
(73) Proprietor: Glengowan B.V., 1217 BG Hilversum (NL)
(72) Inventor: VAN FURTH, Wouter, NL-3633 CV Vreeland (NL); DE VRIES, Luc, NL-7478 AB Diepenheim (NL); WINDELER, Eric, Toronto, Ontario M4N 1J3 (CA)
(74) Representative: Clarkson, Paul Magnus
(86) International application number: PCT/EP2005/003476
(87) International publication number: WO 2005/094698

(56) References cited:
- EP-A- 0 351 165
- DE-C- 759 986
- FR-A- 2 474 303
- US-A- 1 359 164
- US-A- 2 504 202
- US-A- 3 878 848
- US-A- 5 342 375
- US-A- 5 405 353
- US-A- 5 490 858
- US-A- 5 897 571
- US-A1- 2003 045 833

## Description

### FIELD OF THE INVENTION

The invention relates to a surgical instrument to be used for suturing tissue while reducing the possibility of needle perforation accidents.

### BACKGROUND

Standard suturing instruments and techniques present significant risks to both patient and surgeon by way of possible glove perforation accidents in which a suture needle penetrates the surgeon's glove. Such perforation accidents may allow pathogenic organisms such as, but not limited to, the hepatitis virus B, the hepatitis virus C and the human immunodeficiency virus (HIV) to be transmitted from the patient to the practitioner.

Conversely, a perforation accident may cause a break in the sterile barrier between practitioner and patient, which increases the risk of the patient's wound becoming infected.

One approach to help avoid this problem involves the use of surgical forceps of the kind described in U.S. Patent Application US 2003/0045833 A1. The surgical forceps described in that application has near the distal end at the outside of an arm of the forceps a flexible material that can be used for manipulating a surgical needle during suturing in order to attempt to prevent needle perforation accidents.

Among the drawbacks of these and other surgical forceps is that when suturing using a surgical needle and a suture attached thereto, the tissue may sustain damage. This is a particular liability where delicate tissue is concerned through which it is difficult to pass the surgical needle without causing tissue damage. The point of the surgical needle initially pushes the tissue forward to subsequently lance it, which causes damage to the tissue. Moreover, in the surgical forceps described in the above application, the placement of the flexible material on the outside of the arm requires release of the tissue in order to receive the needle. Alternatively, the instrument may be pushed further into the wound thereby increasing the likelihood of damaging the tissue and/or previously tied sutures.

Thus, there remains a need for suturing forceps, which minimize the risk of glove perforation accidents and tissue damage.

### SUMMARY OF THF INVENTION

The present invention , as claimed in claim 1 with preferred embodiments disclosed in the dependent claims, is directed to a surgical instrument (suturing forceps) useful in conventional surgery, endoscopic and robotic surgery. The instrument comprises a first arm and a second arm that are connected at a proximal end (optionally spring-connected) so as to bias the arms in an open configuration and which define a space between them which can be reduced or increased. At a distal end, the arms can be moved towards each other thereby reducing the space between the arms. The instrument further comprises a surgical needle-receiving and affixing portion herein referred to as a "bullet" at the distal end of at least the first arm and/or the second arm, at an inside and/or lower side of the distal end of an arm. Preferably the bullet forms part of the tissue-gripping surface of the forceps. The bullet may be removable from the instrument.

The invention is further directed to the forceps described above comprising an arm manipulating means by which one arm of the instrument, preferably the arm lacking the bullet may move away from the surgical field during the suturing procedure. Specifically, the manipulated arm is removed from the path defined by the movement of the surgical needle during suturing, which is itself defined by the curvature of the needle. Such manipulation of an arm avoids contact with the tissue being sutured thereby preventing tissue damage and facilitating the suturing process.

This may be accomplished using a variety of means including, for example, using a hinge mechanism fixed at the proximal or distal end of at least one arm of the instrument, as described in more detail below. The bullet can be placed at the distal end of the first arm and/or at the distal end of the hinge mechanism. The hinge is designed to provide a means for drawing the distal end of one of the arms away from the surgical field (preferably the arm opposite the bullet) as the distance between the distal ends of the arms increases. Conversely, as the distance between the distal ends of the arms decreases, the distal end of the arm acted upon by the hinge mechanism extends so that the arms become of similar length as the distal ends of the arms make contact with each other.

It is further disclosed a method for suturing tissue using the instrument of the present invention comprising the steps of 1) securing and supporting a first area of tissue to be sutured with the distal ends of the instrument, 2) securing a surgical needle and a suture material attached thereto with a needle holding tool, 3) piercing the first area of tissue to be sutured with the needle using the bullet to support the tissue, 4) passing the needle through the tissue into or onto the bullet, 5) releasing the needle from the needle holding tool, 6) releasing the first area of tissue secured by the distal ends of the instrument, 7) guiding the affixed needle with the instrument following the curvature of the needle, 8) removing the needle from the bullet with the needle holding tool, and 9) repeating steps 1-8 on a second area of tissue to be sutured to the first area of the lesion whereupon the suture passing through the first and second areas of tissue is tied in a knot. Alternatively, prior to the bullet receiving and affixing the surgical needle, the surgical needle in one movement may pierce the first and second areas of tissue to be sutured. The suture is knotted by a force applied by the needle holding tool pulling on the end of the suture and a second pulling force applied by the instrument to which the surgical needle is affixed.

According to an important aspect of the present invention, the bullet is provided as or on a disposable item that can be affixed to a standard medical forceps. In one important embodiment, the bullet is provided on a disposable forceps-like device that can itself be affixed to a standard medical forceps or other suitable manipulative device. Of importance, the disposable portion is preferably embodied as a single use component. Reuse may be prevented by ensuring that a connection, formed between the bullet or its holder and the forceps or manipulator, is broken once the connection is opened. The bullet may then be disposed of together with the suture needle on finishing the suture procedure.

According to a yet further aspect of the invention, the bullet is provided as part of a surgical system comprising all those items required for performing suture. The surgical system may comprise a bullet as herein described, with or without a holder, together with a surgical needle and suture. An appropriate dispensing package may be provided for conveniently dispensing a number of needles and sutures to the surgeon during the suturing procedure. In particular the suture may be of the atraumatic type in which an eyeless needle and suture are combined. Such a system may facilitate suitable presentation of, and disposal of, said forceps and or bullet and or surgical needle and suture, and or other surgical sharp implements such as a scalpel blade, in such a manner to facilitate the surgical and suturing process, and to eliminate further the possibility of percutaneous injuries. The system will comprise some or all aspects of the items described herein, and related packaging to facilitate the effective use of the system.

Further advantages of the invention will be appreciated with reference to the appended claims.

### BRIEF DESCRIPTION OF THE DRAWINGS

The invention will now be further elucidated by way of exemplary embodiments that form no limitation to the appended claims, and with reference to the following drawings.

In the drawings:
Figure 1 shows a perspective view of a surgical instrument according to the invention;
Figure 2 shows a side elevation of the surgical forceps shown in Figure 1;
Figure 3 shows the surgical forceps depicted in Figure 1 wherein a holder with a bullet is detached from the forceps;
Figures 4-7 show several successive stages of using the surgical forceps according to the invention while suturing tissue;
Figure 8 shows a diagram illustrating the wire mesh embodiment of the bullet;
Figure 9 shows a diagram illustrating the form lock embodiment of the bullet;
Figure 10 shows a diagram illustrating the saloon doors embodiment of the bullet;
Figure 11 shows a perspective view of the preferred embodiment of the surgical forceps with a double hinge mechanism in the closed position;
Figure 12 shows a perspective view of the preferred embodiment of the surgical forceps with a double hinge mechanism in the open position;
Figure 13 shows perspective views of the preferred embodiment of the surgical forceps with a double hinge in both open and closed positions;
Figure 14: shows a diagram illustrating the spring enforced sliding arm embodiment of the surgical forceps;
Figure 15 shows a diagram illustrating the flipping bullet embodiment of the surgical forceps;
Figure 16 shows a diagram illustrating the accentric axis embodiment of the surgical forceps;
Figure 17 shows a diagram illustrating the double spring embodiment of the surgical forceps;
Figure 18 shows a schematic view of a mini-forceps embodiment of the surgical forceps in open and closed positions.
Figure 19 shows a perspective view illustrating the mini-forceps embodiment; and
Figure 20 shows a schematic view of the attachment of the mini-forceps to a surgical instrument.

### DETAILED DESCRIPTION OF THF INVENTION

The invention is directed to a surgical instrument (suturing forceps), with which needle perforation accidents can be avoided, tissue damage during suturing can be reduced and which allows suturing to be performed more easily.

The following description is made by way of example and is not intended to limit the invention as set out in the appended claims.

The forceps of the present invention comprises at least a first and second arm that are connected at one end and which may be biased, for example, by a spring means, in an open position and which defines a space between them, which can be reduced or increased. The instrument also comprises a needle receiving and affixing bullet that is preferably positioned at the distal end, at an inside and/or lower side of the end of an arm. The term "open" in the context of the present invention refers to the position wherein the distal ends of the two arms are apart. The term "closed" refers to the position wherein the distal ends of the two arms are in close proximity or touching. The term "form lock" refers to the properties of a bullet embodiment whereby the deformation of a bullet that is pierced by a surgical needle results in a pressure exerted on the surgical needle from the resistance of the bullet to return to its original form thereby affixing (locking) the surgical needle to the bullet.

When suturing tissue using a suture and surgical needle, the forceps of the present invention makes it possible to control the suturing process in such a manner that immediately after the point of the surgical needle has pierced the tissue, it is able to pass into the bullet where it is retained until removal by the surgeon, preferably with the use of a surgical needle holder. This allows the surgical needle to be manipulated safely during suturing without touching the needle with the hands, thereby reducing the possibility of needle perforation accidents.

The bullet also provides support for the portion of tissue being sutured by using the forceps of the present invention to effectively avoid tissue damage because the bullet provides a counter pressure against the pressure of the needle supporting the tissue to be sutured thereby minimizing tissue stretch. Furthermore, the suturing operation can be continued using the forceps to manipulate the needle, without the necessity of either manually touching the needle or using a needle holding instrument.

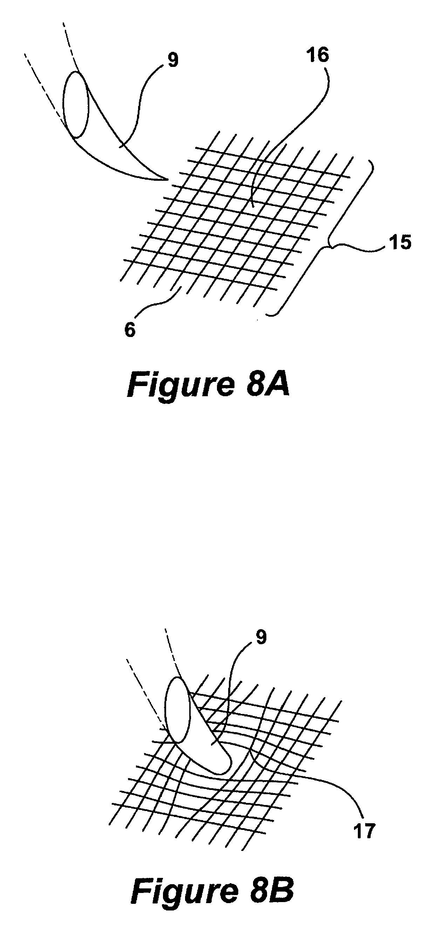

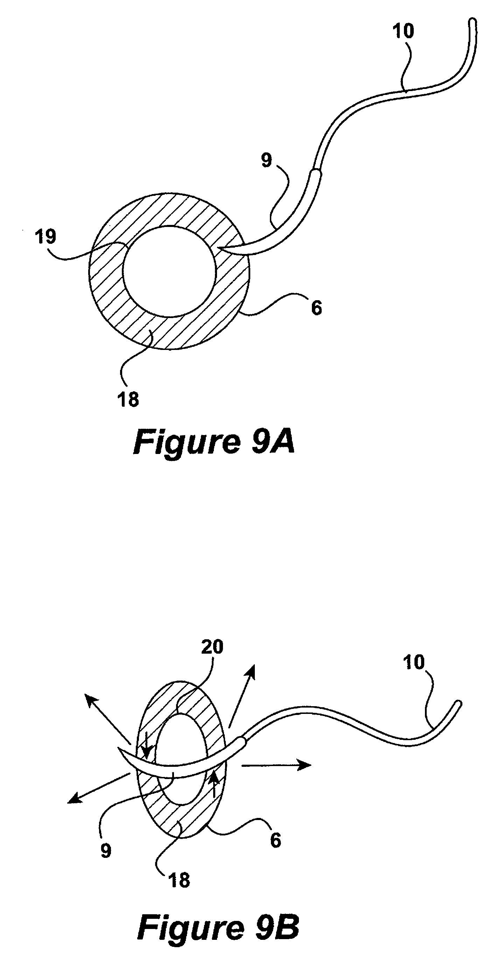

The bullet may be comprised of any material or designed in any way such that it is capable of receiving and removably affixing or retaining a surgical needle. For example, the material may be pierceable such as synthetic rubber or wire mesh. An example of a bullet comprised of wire mesh is illustrated in Figure 8. The bullet may also be comprised of a hollow synthetic material such that when penetrated, the pressure of the needle penetrating the first wall of the bullet deforms the bullet, and this pressure, together with the pressure created by the penetration and subsequent deformation of the second wall creates significant pressure on the needle creating a "form lock," enhancing the bullet's grip on the needle. An example of the form lock embodiment of the bullet is illustrated in Figure 9. Alternatively the hollow space may be filled with a substance such as a gel that may contribute to "locking" a surgical needle. The bullet may also be comprised of a material with magnetic properties suitable for receiving and retaining a surgical needle or any other receiving and retaining means as with an adhesive.

Other means such as inducible gripping mechanisms may also be used to receive and removably affix a needle. For example, the bullet may be designed with an opening that allows a surgical needle to be inserted frictionlessly into the bullet such that when the distal ends of the arms are apart, a plunger mechanism which is dependent on the distance between the distal ends of the arms is activated and pushes on the part of the needle that is through the opening. The pushing force of the plunger acts as a guillotine and results in a grip on the needle thereby affixing the needle to the bullet. Conversely, when the distal ends of the arms are in close proximity, the plunger mechanism responds by retracting the plunger, removing the pressure on the needle, and the needle is released. Alternatively, the plunger mechanism may be independent of the distance between the distal ends of the forceps and may be activated manually by the surgeon. The bullet may also be comprised of a substance, for example, a soft ge1, that receives a surgical needle which by applying or inducing a change in temperature at the site of the bullet, for example by using a laser, causes it to harden thereby fixing the surgical needle that can be released by again applying or inducing a change in temperature at the site of the bullet for example by extinguishing the laser light The bullet of the above-mentioned embodiments may be comprised of a biodegradable material in the event that if any part of the bullet falls into the wound, no additional harm would be caused to the patient, as the bullet would harmlessly dissolve in the body.

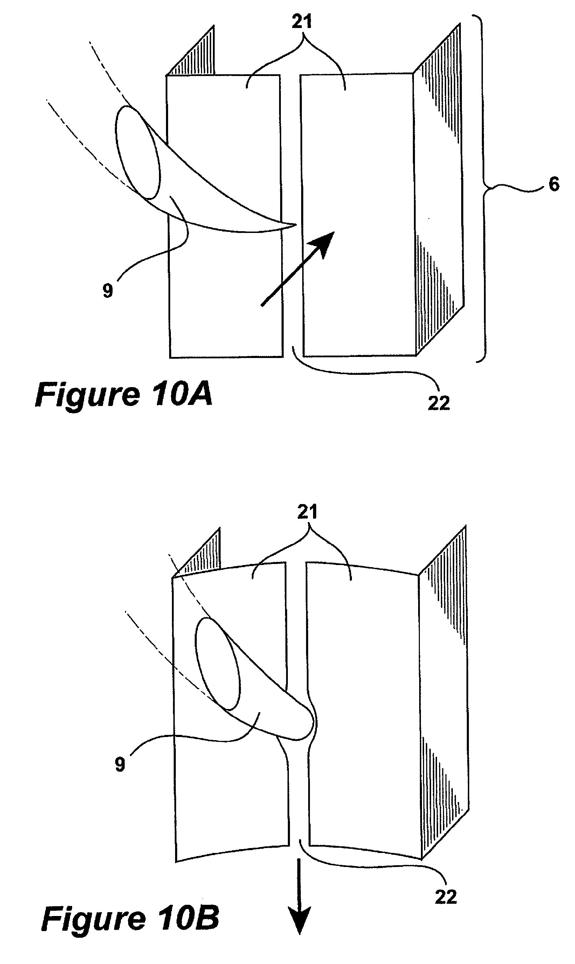

The bullet may also be designed to have a narrow slit into which a surgical needle is guided such that when the forceps are manipulated to pull on the needle, flaps of the slit close thereby affixing the needle to the bullet. The needle is released by pulling or pushing it in the direction of the flaps thereby opening the flaps. Such an embodiment may be referred to as a "saloon door" mechanism. An example of this embodiment is illustrated in Figure 10.

In a further aspect of the invention, the bullet may be "loaded" with an electric or other charge or with receptors to guide the needle to the bullet such that the needle is controlled and gripped more easily.

One form of inducible gripping means may be actuated by the pressure on the bullet due to the normal forces that are on the bullet when used to suture. When the forceps is closed, e.g. on holding tissue, the bullet is deformed by the applied closing pressure and thereby transformed to a more open configuration that allows the needle to be inserted or extracted more easily. When the forceps is opened, the bullet returns to its natural form and in that form the resistance on the needle is increased, making it more difficult to pull the needle out or pierce it in, however facilitating manipulation of the needle by manipulation of the forceps. This considerably aids the normal actions during suture such as when the needle is pulled through the tissue.

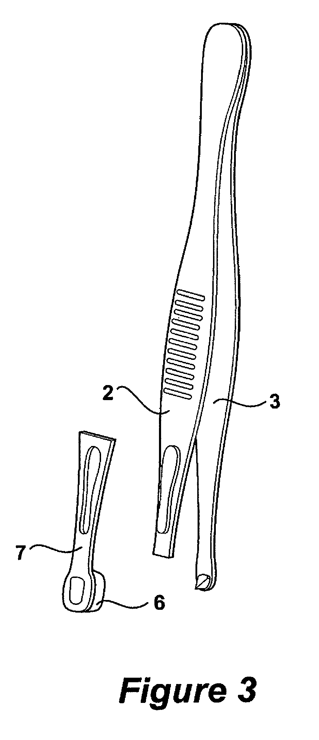

In a further aspect of the invention the bullet is provided on a holder that is detachably placed on at least one arm as exemplified herein. The holder with the bullet may thus be a disposable component that can be supplied sterile, while the forceps upon which the holder is placed may be retained and sterilized from case to case. The holder with the bullet is designed to be removably placed on forceps of the kind illustrated in Figure 3.

In using the present invention there is a moment in suturing in which the surgeon pulls the needle (that is already fixed in the bullet) through the tissue. In order to do so without harming the tissue, the surgeon guides the forceps following the curvature of the needle. By doing so, the arm opposite to the arm with the bullet to which the needle is affixed can touch or even get stuck in the tissue. Providing a means for moving the opposite arm away from the surgical field when the distal ends of the two arms are apart alleviates this problem.

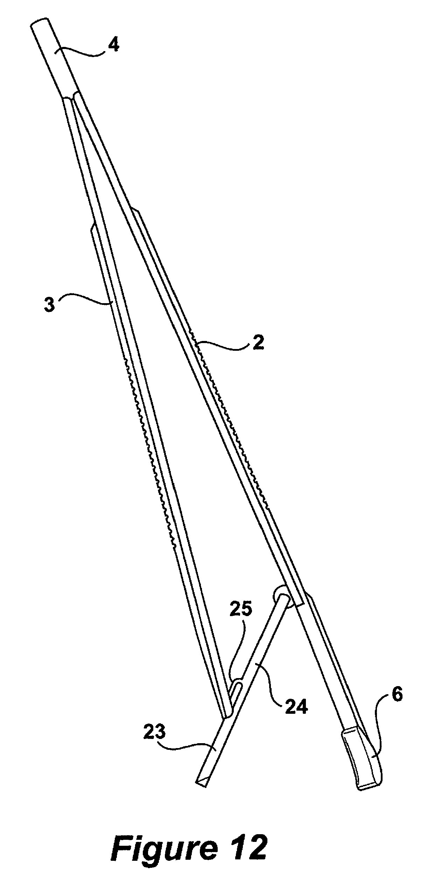

To this end, a preferred embodiment of the invention is comprised of the two arms of the forceps being of unequal length with a hinge mechanism, which is comprised of a hinge and a lever, attached to the shorter arm that is opposite the bullet. When the forceps are in a closed position, the distal end of lever extends to contact the distal end of the arm to which the bullet is attached. When in the open position, the lever retracts and is no longer in the way of making a circular motion with the forceps to pull the needle through the tissue along the curvature of the needle. Such an embodiment may be referred to as "double-hinge." An example of this embodiment is illustrated in Figure 12.

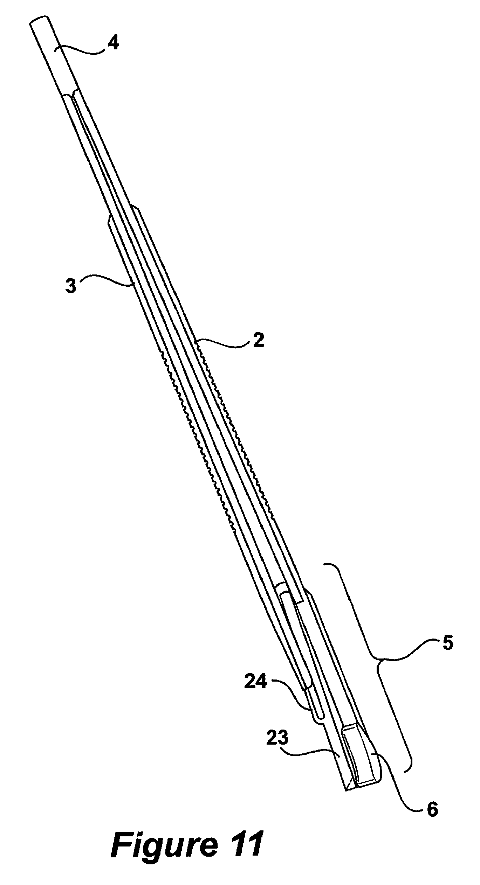

For this embodiment, the hinge mechanism is comprised of a hinge that is medially fixed to a lever. The hinge is also fixed at the distal end of the shorter arm opposite the bullet such that the distal end of the lever acts as an-extension of the shorter arm. The proximal end of the lever is slidably disposed along the inside of the longer arm. Examples of this embodiment are illustrated in Figures 11, 12 and 13.

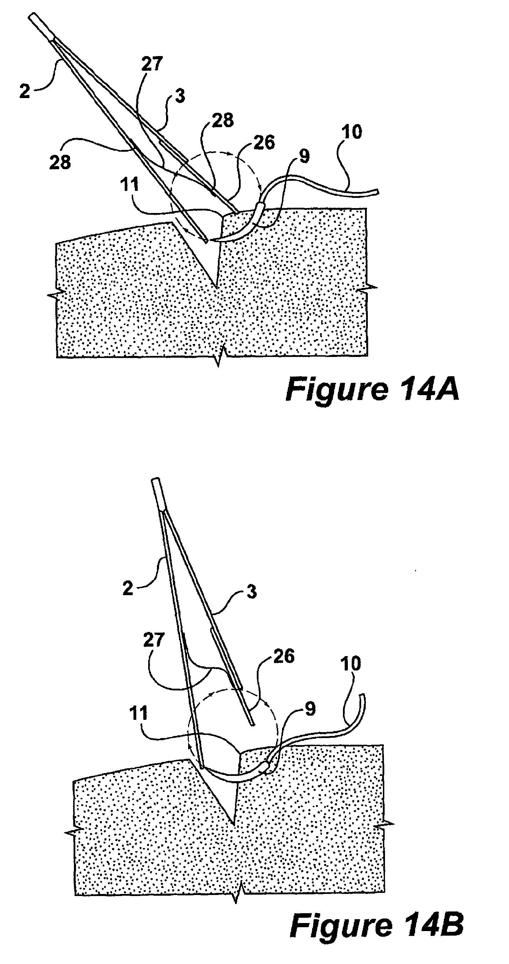

In an alternate embodiment of the invention, the hinge mechanism is in the form of a spring medially fixed to a sliding lever. The spring opens the forceps and by motion of the sliding lever makes the distal end of the arm that is to be moved away (i.e. the arm opposite the arm with the bullet) slide in the proximal direction when the forceps are open. When the forceps are closed, the sliding lever slides towards the distal end of the forceps to enable sufficient grip of the tissue. Such an embodiment may be referred to as "spring enforced sliding arm." An example of this embodiment is illustrated in Figure 14.

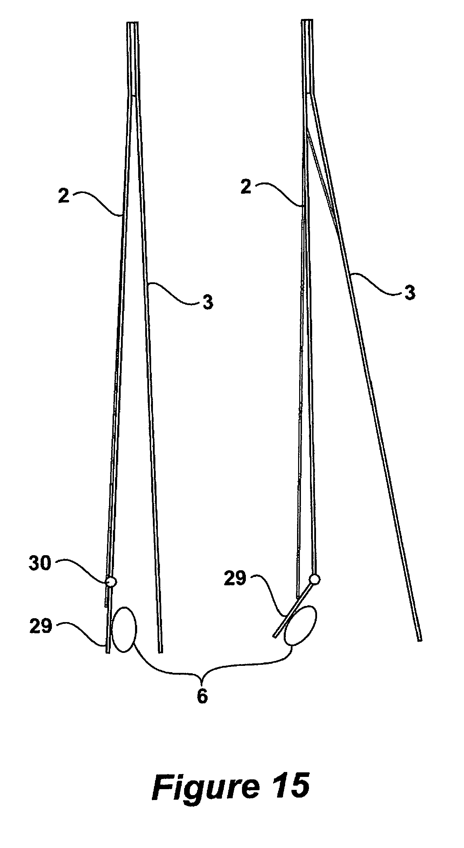

In an alternate embodiment of the invention, the hinge mechanism is fixed to the distal end of the shorter arm of the forceps. Furthermore, the bullet is placed at the distal end of the hinge mechanism. Spring-forced movement of the hinge flips it away from the longer arm of the forceps and allows the surgeon to pull the surgical needle through, along the natural needle path, without damaging the tissue. Such an embodiment may be referred to as "flipping bullet." An example of this embodiment is illustrated in Figure 15.

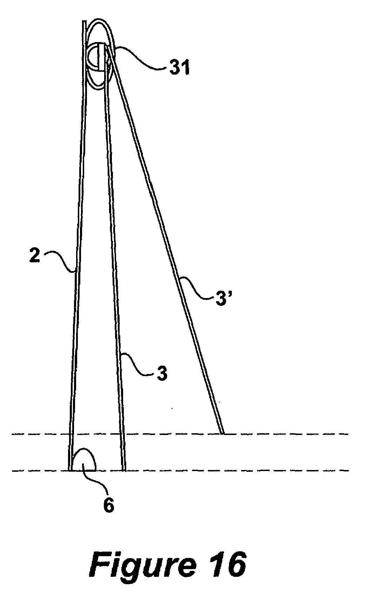

In an additional embodiment of the invention, an elliptical hinge mechanism at the proximal end of the forceps may be used in which the point of rotation in the proximal end makes a translating movement at the same time as the rotating movement takes place. This design "shortens" the arm that is opposite the bullet when the forceps are opened. Such an embodiment may be referred to as "accentric axis." An example of this embodiment is illustrated in Figure 16.

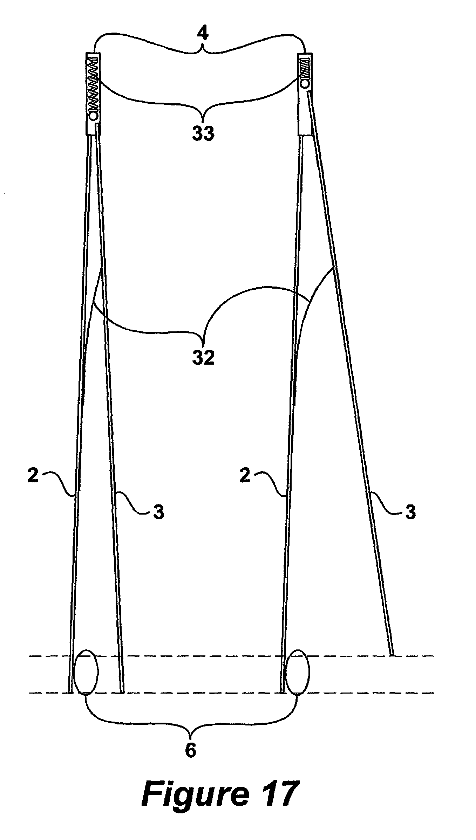

In an additional embodiment of the invention, the hinge mechanism is comprised of a double spring. A first spring holds the two arms apart in the open position in which the arm opposite that which holds the bullet is shorter. When the first spring is engaged and the distal ends of the two arms are brought together, a second spring located at the proximal end of the instrument and fixed to the shorter arm is also engaged and extends the shorter arm such that the distal ends of the two arms meet when the forceps are in the closed position. Such an embodiment may be referred to as "double spring." An example of this embodiment is illustrated in Figure 17. Other means by which to accomplish shortening of the arm of the forceps will be readily apparent to one of ordinary skill in the art.

Identical reference numerals used in the figures refer to similar parts.

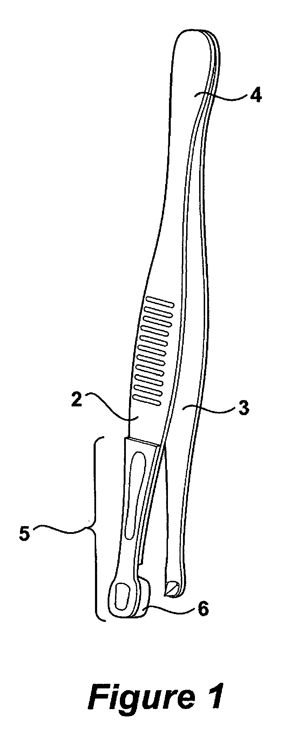

Referring first to Figure 1, where reference numeral 1 indicates the surgical forceps according to the invention.

These surgical forceps 1 are suitable to be used for suturing tissue and comprise a first forceps arm 2 and a second forceps arm 3, spring-connected at a proximal end 4, i.e. the end which during the manipulation of the forceps 1 lies in the hand and which arms define a space between them which an be reduced and increased.

At a distal end 5, the first forceps arm 2 and the second forceps arm 3 can be moved toward each other.

Figure 1 further shows that the first forceps arm 2 is provided with a bullet 6. This bullet 6, in its preferred embodiment, is comprised of a needle receiving and retaining material such an elastomeric material which is suitable to be pierced with a surgical needle and which removably retains the needle until removed by the surgeon, as will be further explained below.

The bullet may also be provided on the second arm 3 or, as the case may be, only on the second arm 3. Within the framework of the invention, however, at least one of the forceps arms 2, 3 must be provided with a bullet 6.

As Figure 1 shows, the bullet 6 is positioned close to or at the distal end 5, at an inside or lower side of the end of the first forceps arm 2.

The bullet 6 in one of its embodiments is preferably designed to be able to receive and affix a surgical needle pierced therein. A material to be used as the bullet 6 is suitably a synthetic material, for example synthetic rubber or other elastomeric material. Advantageously, the bullet 6 together with the end of the arm upon which it is placed define a space between the first arm 2 and the second arm 3 which can be reduced or increased. The fabrication of this is well known to the person skilled in the art and requires no further elucidation.

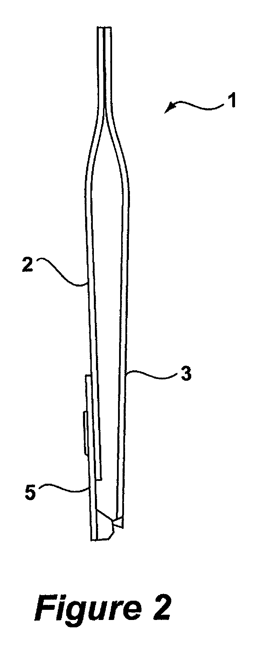

Figure 2 shows a side elevation of the surgical forceps 1 according to the invention wherein the first forceps arm 2 and the second forceps arm 3 are moved toward each other. In this embodiment it is schematically shown that the bullet may slidably extend from the arm 2 on releasing the grip on the tissue.

Figure 3 shows that the bullet 6 is provided on a holder 7 that is detachable from but, as in the illustrated case, can also be detachably placed on the first forceps arm 2. Any means of attachment and detachment may be used including that illustrated in Figure 3, screw on and off attachment means, clip on, luer-lock and others.

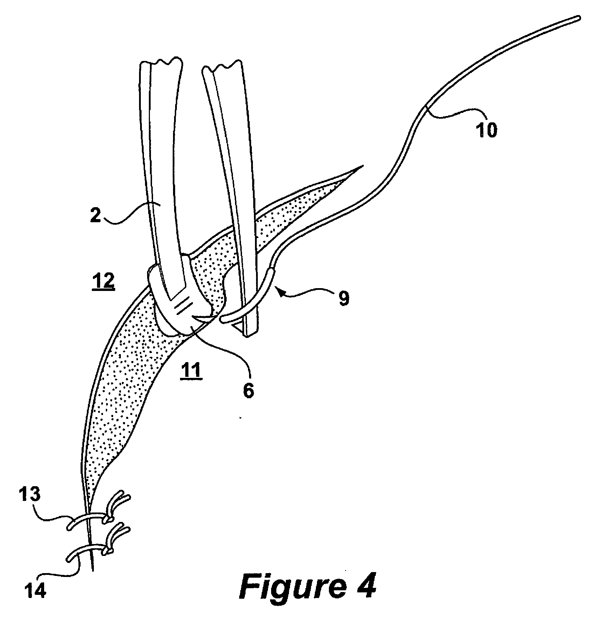

The use of the surgical forceps 1 according to the invention may conveniently be explained by way of a series of successive steps illustrated in the Figures 4-7, showing the use of the surgical forceps 1 according to the invention for suturing tissue.

Figure 4 shows a first step, wherein by means of a needle-holding tool (not shown) a surgical needle 9, attached to which is a suture 10, pierces a first tissue portion 11 in order to join this first tissue portion 11 with a second tissue portion 12.

Reference numerals 13 and 14 indicate two sutures made previously through the first and second tissue portions 11 and 12.

Figure 4 shows clearly that the first forceps arm 2, which at the inside distal end is provided with a bullet 6, serves to support the first tissue portion 11 through which the suture 10 is passed. In this way the surgical forceps 1 according to the invention are able to effectively support the first tissue portion 11 so as to avoid damage to this first tissue portion 11, while simultaneously a point of the surgical needle 9 is able to pass into the bullet 6 in order to receive and affix the surgical needle 9 therein.

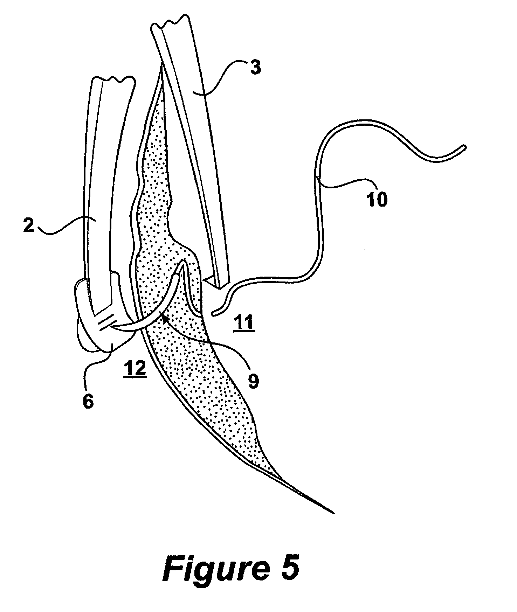

Figure 5 subsequently shows that the surgical needle 9 can be passed further through the first tissue portion 11 by employing the surgical forceps 1 in accordance with the invention.

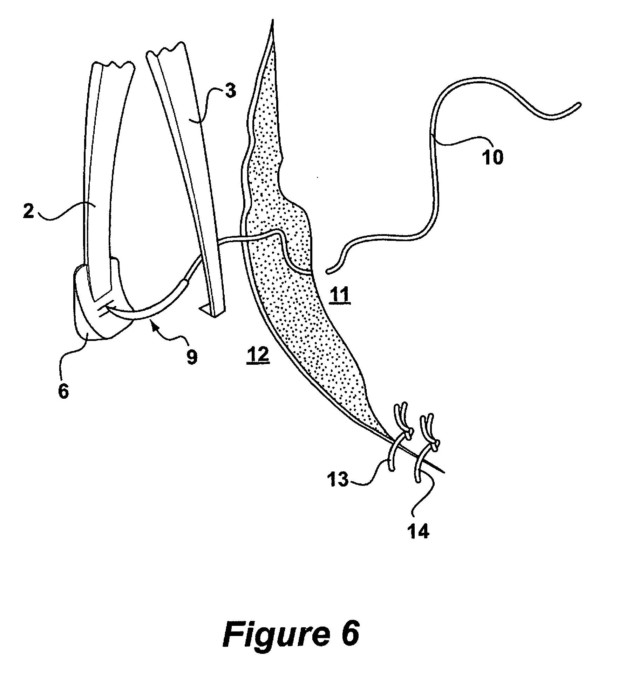

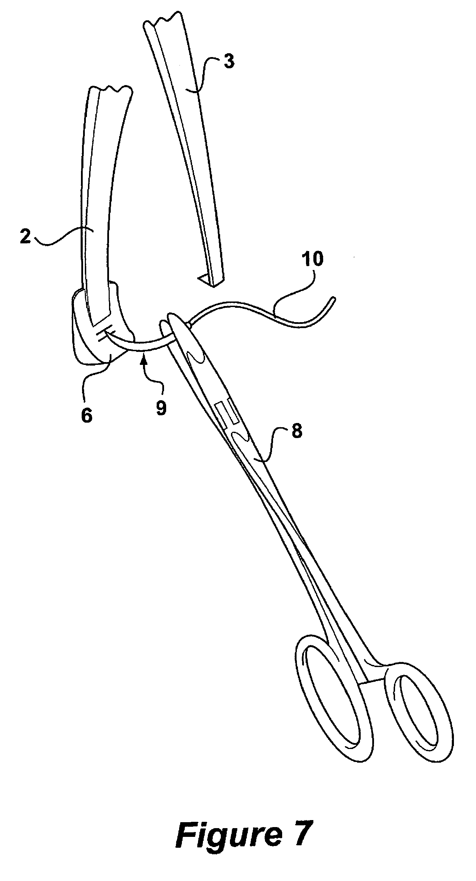

Figure 6 subsequently shows that the surgical needle 9, with the suture 10 attached thereto, is in an advanced stage of its passage through the first tissue portion 11 and as Figure 7 further shows, that the surgical needle 9 thus becomes available again for manipulation by using a needle-holding tool 8.

Figures 8-10 show embodiments of various mechanisms by which the bullet 6 may receive and affix a surgical needle 9. Although these bullets are disclosed in combination with forceps according to the present invention, it is also contemplated that such bullets may be affixed to other implements and may themselves be the subject of inventions.

Figure 8 shows a wire mesh embodiment of the bullet 6 wherein a tightly woven mesh with wires 15 that can slide in relation to each other, with at some intervals no sliding knots between wires. The surgical needle 9 is inserted in one of the pores 16 resulting in displacement of the wires until a non-moving corner 17 is encountered. The interval of the non-moving corner 17 assists in the grip on the needle 9.

Figure 9 shows a "form lock" embodiment wherein a bullet 6 is comprised of a layer 18 of synthetic rubber material with a hollow core. The bullet may be of any shape. The act of inserting a surgical needle 9 through the layer 18 and subsequently through an open space 19 and back into the layer 18 of the bullet 6 causes a deformation of the bullet 6. The physical dynamics of the bullet 6 trying to return to its neutral shape, due to its material memory, causes increased pressure to be borne on the needle 9, thereby increasing the grip the bullet 6 has on the needle 9. Alternative versions including a plurality of open spaces such as a foam may also be considered.

Figure 10 shows a "saloon doors" embodiment of the bullet 6 wherein the surgical needle 9 is stuck between two pieces of material or flaps 21 that have a very narrow slit 22 in between. The needle 9 is guided to go between the two flaps 21, resulting in "opening the saloon doors." When the forceps are manipulated to pull the surgical needle 9 out of the tissue, the flaps 21 close, resulting in a grip on the surgical needle 9 because of the additional space the needle 9 occupies between the flaps 21. The greater the pulling force applied, the stronger the grip on the needle 9 because of the friction between the needle 9 on the flaps 21 forces the flaps to close further. After the needle 9 is pulled through the tissue, the needle 9 is released by pulling or pushing it in the direction the flaps 21 open.

The remaining figures address the potential problem encountered in suturing in which the surgeon wants to pull the needle (that is affixed to the bullet) through the tissue. In order to do so without harming the tissue, the surgeon will want to guide the forceps following the curvature of the needle. By doing so, the arm opposite the arm with the flexible material can touch or even get stuck in the tissue.

Figures 11-13 illustrate the surgical forceps with a double hinge mechanism. In Figure 11, the instrument is comprised of a longer first arm 2 and a shorter second arm 3 with a hinge mechanism that includes a lever 23 and a hinge 24 fixed to the distal end 5 of the second arm 3. When combined with the lever 23, the distal end of lever 23 contacts the distal end of the first arm 2 when the instrument is in the closed position.

Figure 12 shows the surgical forceps 1 with the hinge mechanism comprised of a lever 23 and a hinge 24 in the open position. A spring 25 may be used to enforce the open position when the forceps are not engaged.

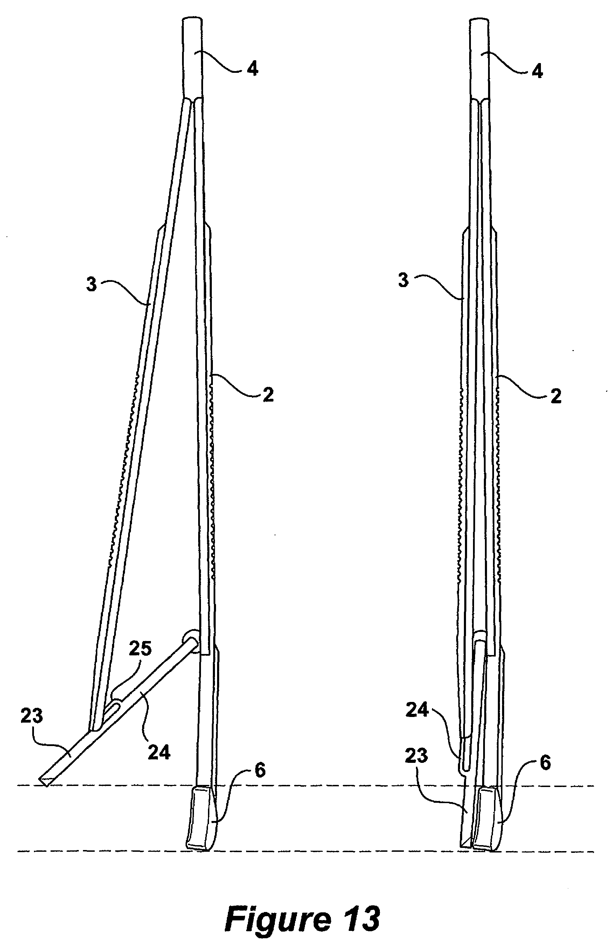

Figure 13 shows the surgical forceps 1 in both the open and closed positions. Note the difference in length of the second arm 3 combined with the lever 23 along the axis of the forceps when in open and closed positions. In the open position, the lever 23 is out of the way when making a circular motion with the forceps to pull a needle (not shown) through tissue along the curvature of the needle thereby avoiding unwanted contact with and/or damage to the tissue.

Figure 14 shows the spring enforced sliding arm embodiment of the surgical forceps 1 wherein the second arm 3 is shorter than the first arm 2. The second arm 3 has attached to it at the distal end a sliding lever 26. A spring 27 is fixed to a medial region 28 of the first arm 2 and the sliding lever 26 such that it makes the distal end of the sliding lever 26 slide in the proximal direction when the forceps are in the open position. When the forceps are closed, the sliding lever 26 slides towards the distal end of the instrument to enable sufficient grip of the first tissue portion 11 to be sutured.

Figure 15 illustrates the flipping bullet embodiment of the invention wherein a short arm 29 to which a bullet 6 is attached is fixed at the distal end of the first arm 2 by a hinge 30. The first arm 2 is shorter than the second arm 3, but in the closed position, the distal end of the short arm 29 to which the bullet 6 is attached touches the distal end of the second arm 3. Spring-forced movement of the hinge 30 that the surgeon can manipulate flips the short arm 29 away from the longer second arm 3 of the forceps and allows the surgeon to pull the surgical needle (not shown) through, along the natural needle path, without damaging the tissue.

Figure 16 illustrates the accentric axis embodiment of the invention wherein an elliptical hinge mechanism 31 in which the point of rotation in the proximal end of the forceps makes a translating movement at the same time as the rotating movement takes place. Under this design, when the forceps are in the closed position, the distal end of the second arm 3 extends such that it meets the distal end of the first arm 2. In the open position, the elliptical hinge mechanism 31 shortens the second arm 3', thereby avoiding damage to the tissue.

Figure 17 illustrates the double spring embodiment of the invention wherein the second arm 3 that is opposite the bullet 6 is retracted and extended by a first spring 32 that holds the first arm 2 and second arm 3 apart in the open position in which the second arm 3 is shorter than the first arm 2. When the first spring 32 is engaged and the distal ends of the two arms are brought together, a second spring 33 located at the proximal end 4 of the forceps and fixed to the shorter second arm 3 is also engaged and extends the shorter second arm 3 such that the distal ends of the first arm 2 and second arm 3 meet when the instrument is in the closed position.

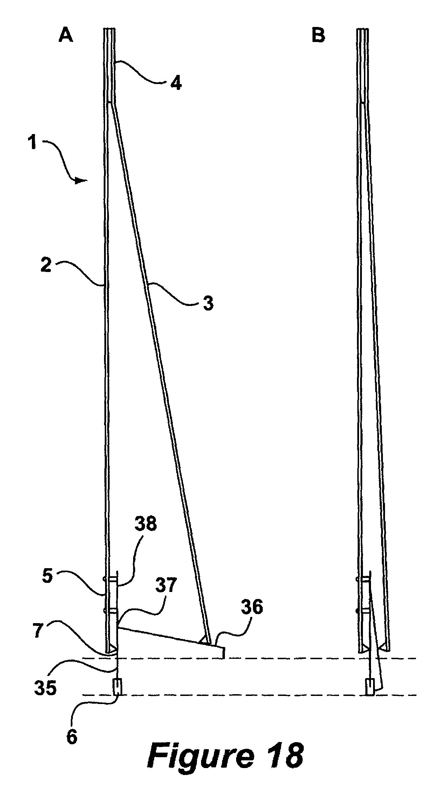

Figures 18 A, B 19 and 20 illustrate a mini-forceps embodiment of the present invention which operates in a similar manner to the double hinge mechanism of Figures 11 - 13. It has the additional advantage that the holder 7 on which the bullet 6 is mounted may be formed as a disposable item for connection to a standard medical forceps or other suitable holder/actuator.

Figure 18A shows a schematic view of the mini forceps embodiment in the open position. According to Figure 18A, a standard surgical forceps 1 comprises a first arm 2 and a second arm 3, spring-connected together at a proximal end 4. A bullet holder 7 carrying bullet 6 is releasably connected to the distal end 5 of the first arm 2. The holder 7 is formed as a mini-forceps and comprises a first member 35 and second member 36 joined together at their proximal ends by a hinge 37. The bullet 6 is provided at the distal tip of the first member 35. A connecting member 38 extends proximally from the first member 35 for connection to the forceps 1.

Figure 18B shows a schematic view of the mini forceps embodiment in closed position. As can be seen from the figure, the limited length of the first and second members 35, 36 relative to the arms 2, 3 ensures that the angular movement of the mini-forceps 7 is considerably greater than that of the forceps 1 on movement between closed and open positions. This increased angular movement ensures that in use, the second member 36 is distanced from the path of movement of the surgical needle 9.

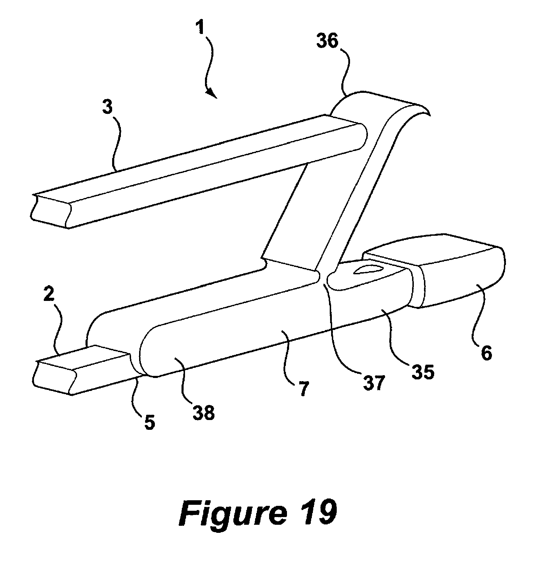

Figure 19 shows a perspective view of the holder 7 which is formed of a suitable medical grade plastics material. The hinge 37 is formed as a living hinge having a resilient bias to an open position. It is evident that alternative materials could be used for forming the holder and hinge and that its construction as a disposable device is merely optional. Furthermore, various connecting mechanisms may be envisaged for attaching the connecting member 38 to the forceps 1.

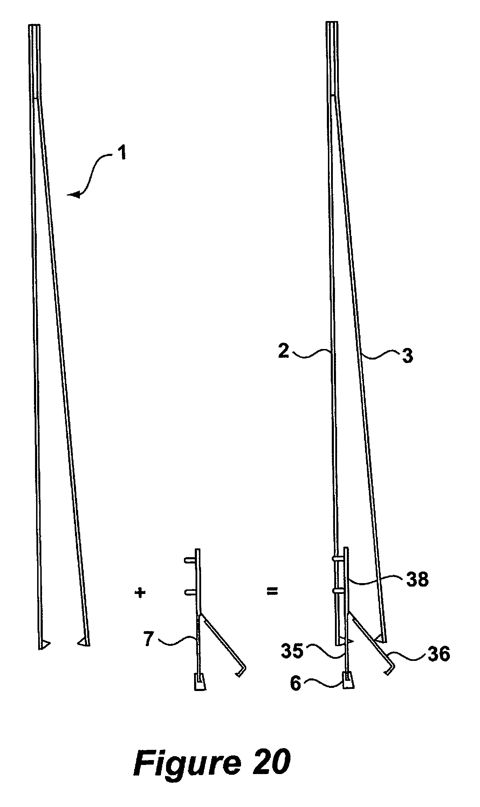

Figure 20 shows a schematic view of the mini forceps embodiment illustrating how the holder (mini forceps) 7 may be connected to e.g. a standard medical forceps 1 to form a combined instrument.

It is also apparent that the forceps according to the present invention is not only useful in "open surgery," its advantages may be exploited in endoscopic surgical procedures or in combination with other endoscopic tools wherein a single arm with a bullet attached at a distal end is employed. The bullet simultaneously supports the tissue to be pierced with a surgical needle and is capable of receiving and affixing the needle thereafter so that the needle can be manipulated with the instrument.

## Claims

1. A forceps (1) to be used for suturing tissue (11), comprising a first arm (2) and a second arm (3) that are connected at a proximal end (4) and are moveable toward and away from one another and which define a space between said arms which can be reduced or increased to grip the tissue, said forceps further comprising a bullet (6), placed at a distal end (5) of said first arm and/or said second arm, at an inside and/or lower side of the end of said arm, to receive and affix a surgical needle (9) while gripping the tissue.

2. The forceps of claim 1, wherein said forceps further comprises a spring connection (25) said spring connection biasing said first arm and said second arm of said forceps in an open position.

3. The forceps according to any preceding claim, wherein said bullet is suitable for being pierced with said surgical needle.

4. The forceps according to any preceding claim, wherein said bullet is comprised of elastomeric material.

5. The forceps according to claim 4, wherein said elastomeric material is synthetic rubber.

6. The forceps according to any preceding claim, wherein said bullet has a hollow core surrounded by a pierceable layer (18) for affixing said surgical needle by a form lock, whereby the pierced material exerts a force on the inserted needle as a result of its deformation

7. The forceps according to claim 6, wherein said open space is comprised of a filler material said filler selected from material comprising gels, foams, beads or liquids.

8. The forceps according to any preceding claim, wherein said bullet comprises wire mesh (15).

9. The forceps according to any preceding claim, wherein said bullet comprises a magnetic material.

10. The forceps according to any preceding claim, wherein said bullet comprises an adhesive material.

11. The forceps according to any preceding claim, wherein said bullet comprises a clamp.

12. The forceps according to claim 1, wherein said bullet comprises an inducible gripping mechanism comprising a plunger mechanism wherein said plunger mechanism is activated as said distal ends of said first arm and said second arm move apart toward an open position and where said distal ends of said arms are in close proximity, said plunger mechanism is retracted thereby releasing said needle.

13. The forceps according to claim 11, wherein said plunger mechanism is manually activated independent of said open or said closed positions of said forceps.

14. The forceps according to claim 1 wherein said bullet is comprised of a hollow structure comprised of at least two flaps (21) separated by a narrow slit (22) into which said surgical needle may be guided said flaps being suitable for affixing said surgical needle to said bullet and where said surgical needle is released from said bullet by pulling said surgical needle through.

15. The forceps according to any preceding claim, wherein said bullet is comprised of a material that responds to changes in chemical or physical conditions such as temperature whereby said bullet affixes and releases said surgical needle upon a change in chemical or physical conditions at site of said bullet.

16. The forceps according to any preceding claim, wherein said bullet is placed on at least one arm.

17. The forceps according to any preceding claim, wherein said bullet is provided on a holder (7) that is removable from said arm.

18. The forceps according to any preceding claim, wherein said bullet is comprised of a biodegradable material.

19. The forceps according to any preceding claim, wherein said first and second arms comprise plastics material and said forceps are disposable.

20. The forceps according to claim 19, wherein said first and second arms are joined together by a living hinge (37).

21. The forceps according to any preceding claim, wherein said forceps are provided with an attachment (38) for removable connection to a medical instrument, in particular an arm of a medical forceps.

22. The forceps according to any preceding claim further comprising a mechanism for distancing the second arm from the suture path.

23. The forceps according to claim 22, wherein said forceps further comprises a hinge mechanism suitable for adjusting the length of at least one arm of said forceps along an axis of said forceps with said hinge mechanism fixed at said proximal and/or distal end of at least one arm of said forceps whereby the difference in length along an axis of said forceps between said first arm and said second arm alone or in combination with said hinge mechanism increases as the distance between said distal ends of said arms increases.

24. The forceps of claim 23, wherein said hinge mechanism is comprised of a hinge (24) fixed to a lever (23).

25. The forceps of claim 23 or claim 24, wherein said second arm is shorter in length than said first arm.

26. The forceps according to any of claims 23 to 25, wherein the difference in length along said axis of said forceps between said first arm and said second arm alone or in combination with said hinge mechanism decreases as the distance between said distal ends of said arms decreases.

27. The forceps according to claim 24, wherein said hinge mechanism is fixed at the distal end of said first arm such that a proximal end of said lever is fixed to said hinge wherein a bullet is placed at a distal end of said lever.

28. The forceps according to claim 24, wherein said hinge mechanism is fixed at said distal end of said second arm such that said hinge is fixed at a medial region of said lever wherein said proximal end of said lever is slidably disposed along the inside of said first arm.

29. The forceps according to claim 24, wherein said hinge is medially fixed at said first arm and medially fixed at said lever wherein said lever is parallel to and slidably disposed along said second ann.

30. The forceps according to claim 23, wherein said hinge mechanism is located at said proximal end of said instrument whereby said second arm is shortened as the distance between said distal ends of said arms increases.

31. The forceps according to claim 30, wherein said hinge mechanism comprises an elliptical accentric axis (31) that is continuous with said second arm such that a translating movement extends the length of said second arm as said distal ends of said first and said second arms are in a closed position.

32. The forceps according to claim 30, wherein said hinge mechanism is comprised of a double spring system wherein a first spring (32) is fixed medially between said first arm and second arm biasing said arms in an open position and a second spring (33) fixed at the proximal end of said forceps wherein said second spring is fixed to a proximal end of said second arm said second spring capable of protracting said second arm as said first and said second arms are in a closed position.

33. The forceps according to any preceding claim, wherein said bullet comprises an inducible gripping mechanism actuated by pressure thereon, whereby pressure exerted on the bullet on contact with the tissue allows easy needle entry and removal of said pressure causes an increased grip on the needle.

34. The forceps according to any of claims 1 to 33, wherein said bullet or said holder is connected to the forceps by a single-use connection that is destroyed on disconnection thereof.

35. A surgical system to be used for suturing tissue, comprising a forceps as defined in any of claims 1 to 34, in combination with a surgical needle and suture.

36. The surgical system according to claim 35, wherein the surgical needle and suture are combined as an atraumatic suture.

## Patentansprüche

1. Pinzette (1) zur Verwendung zum Nähen von Gewebe (11), umfassend einen ersten Schenkel (2) und einen zweiten Schenkel (3), die an einem proximalen Ende (4) miteinander verbunden und aufeinander zu und voneinander weg beweglich sind und einen Zwischenraum zwischen den Schenkeln definieren, der reduziert oder erweitert werden kann, um das Gewebe zu greifen, wobei die Pinzette weiterhin eine Fassung (6) umfasst, die sich am distalen Ende (5) des ersten Schenkels und/oder des zweiten Schenkels an der Innenseite und/oder Unterseite des Endes dieses Schenkels befindet, um eine chirurgische Nadel (9) aufzunehmen und zu fixieren, während die Pinzette das Gewebe greift.

2. Pinzette gemäß Anspruch 1, wobei die Pinzette weiterhin eine Federverbindung (25) umfasst, wobei die Federverbindung den ersten Schenkel und den zweiten Schenkel der Pinzette in geöffneter Position unter eine Vorspannung setzt.

3. Pinzette gemäß einem der vorstehenden Ansprüche, wobei die Fassung von der chirurgischen Nadel durchstochen werden kann.

4. Pinzette gemäß einem der vorstehenden Ansprüche, wobei die Fassung aus elastomerem Material besteht.

5. Pinzette gemäß Anspruch 4, wobei es sich bei dem elastomeren Material um synthetischen Kautschuk handelt.

6. Pinzette gemäß einem der vorstehenden Ansprüche, wobei die Fassung einen hohlen Kern, der von einer durchstechbaren Schicht (18) umgeben ist, zum Fixieren der chirurgischen Nadel durch einen Formschluss aufweist, wodurch das durchstochene Material infolge seiner Verformung eine Kraft auf die eingeführte Nadel ausübt.

7. Pinzette gemäß Anspruch 6, wobei der offene Raum aus einem Füllmaterial besteht, wobei das Füllmaterial aus Materialien ausgewählt ist, die Gele, Schaumstoffe, Beads oder Flüssigkeiten umfassen.

8. Pinzette gemäß einem der vorstehenden Ansprüche, wobei die Fassung ein Drahtnetz (15) umfasst.

9. Pinzette gemäß einem der vorstehenden Ansprüche, wobei die Fassung ein magnetisches Material umfasst.

10. Pinzette gemäß einem der vorstehenden Ansprüche, wobei die Fassung ein adhäsives Material umfasst.

11. Pinzette gemäß einem der vorstehenden Ansprüche, wobei die Fassung eine Klemme umfasst.

12. Pinzette gemäß Anspruch 1, wobei die Fassung einen induzierbaren Greifmechanismus umfasst, der einen Stößelmechanismus umfasst, wobei der Stößelmechanismus aktiviert wird, wenn sich die distalen Enden des ersten Schenkels und des zweiten Schenkels auseinander in eine geöffnete Stellung bewegen, und wo sich die distalen Enden der Schenkeln nahe beieinander befinden, der Stößelmechanismus zurückgezogen wird, wodurch die Nadel freigegeben wird.

13. Pinzette gemäß Anspruch 11, wobei der Strißelmechanismus unabhängig von der geöffneten oder geschlossenen Stellung der Pinzette manuell aktiviert wird,

14. Pinzette gemäß Anspruch 1, wobei die Fassung aus einer hohlen Struktur besteht, die aus wenigstens zwei Klappen (21) besteht, die durch einen schmalen Schlitz (22) voneinander getrennt sind, in den die chirurgische Nadel geführt werden kann, wobei die Klappen geeignet sind, die chirurgische Nadel in der Fassung zu fixieren, und wobei die chirurgische Nadel aus der Fassung freigesetzt wird, indem man die chirurgische Nadel hindurchzieht.

15. Pinzette gemäß einem der vorstehenden Ansprüche, wobei die Fassung aus einem Material besteht, das auf Änderungen der chemischen oder physikalischen Bedingungen, wie der Temperatur, reagiert, wodurch die Fassung die chirurgische Nadel nach einer Änderung der chemischen oder physikalischen Bedingungen am Ort der Fassung fixiert bzw. freisetzt.

16. Pinzette gemäß einem der vorstehenden Ansprüche, wobei die Fassung auf wenigstens einen Schenkel gesetzt wird.

17. Pinzette gemäß einem der vorstehenden Ansprüche, wobei sich die Fassung auf einer Haltevorrichtung (7) befindet, die von dem Schenkel abgenommen werden kann.

18. Pinzette gemäß einem der vorstehenden Ansprüche, wobei die Fassung aus einem biologisch abbaubaren Material besteht.

19. Pinzette gemäß einem der vorstehenden Ansprüche, wobei der erste und der zweite Schenkel ein Kunststoffmaterial umfassen und die Pinzette zur einmaligen Verwendung vorgesehen ist.

20. Pinzette gemäß Anspruch 19, wobei der erste und der zweite Schenkel durch ein Filmscharnier (37) miteinander verbunden sind.

21. Pinzette gemäß einem der vorstehenden Ansprüche, wobei die Pinzette mit einer Halterung (38) zur lösbaren Verbindung mit einem medizinischen Instrument, insbesondere einem Schenkel einer medizinischen Pinzette, versehen ist.

22. Pinzette gemäß einem der vorstehenden Ansprüche, die weiterhin einen Mechanismus umfasst, um den zweiten Schenkel vom Weg der Naht wegzuhalten.

23. Pinzette gemäß Anspruch 22, wobei die Pinzette weiterhin einen Gelenkmechanismus umfasst, der geeignet ist, um die Länge wenigstens eines Schenkels der Pinzette entlang einer Achse der Pinzette zu justieren, wobei der Gelenkmechanismus am proximalen und/oder distalen Ende wenigstens eines Schenkels der Pinzette fixiert ist, wobei der Längenunterschied entlang einer Achse der Pinzette zwischen dem ersten Schenkel und dem zweiten Schenkel allein oder in Kombination mit dem Gelenkmechanismus zunimmt, wenn der Abstand zwischen den distalen Enden der Schenkel zunimmt.

24. Pinzette gemäß Anspruch 23, wobei der Gelenkmechanismus aus einem Gelenk (24) besteht, das an einem Hebel (23) fixiert ist.

25. Pinzette gemäß Anspruch 23 oder 24, wobei der zweite Schenkel kürzer ist als der erste Schenkel.

26. Pinzette gemäß einem der Ansprüche 23 bis 25, wobei der Längenunterschied entlang der Achse der Pinzette zwischen dem ersten Schenkel und dem zweiten Schenkel allein oder in Kombination mit dem Gelenkmechanismus abnimmt, wenn der Abstand zwischen den distalen Enden der Schenkel abnimmt.

27. Pinzette gemäß Anspruch 24, wobei der Gelenkmechanismus am distalen Ende des ersten Schenkels fixiert ist, so dass ein proximales Ende des Schenkels an dem Gelenk fixiert ist, wobei eine Fassung am distalen Ende des Hebels platziert ist.

28. Pinzette gemäß Anspruch 24, wobei der Gelenkmechanismus am distalen Ende des zweiten Schenkels fixiert ist, so dass das Gelenk an einem mittleren Bereich des Hebels fixiert ist, wobei das proximale Ende des Hebels so angeordnet ist, dass es entlang der Innenseite des ersten Schenkels gleiten kann.

29. Pinzette gemäß Anspruch 24, wobei das Gelenk mittig an dem ersten Schenkel und mittig an dem Hebel fixiert ist, wobei der Hebel parallel zum zweiten Schenkel so angeordnet ist, dass er an ihm entlang gleiten kann.

30. Pinzette gemäß Anspruch 23, wobei sich der Gelenkmechanismus am proximalen Ende des Instruments befindet, wobei der zweite Schenkel verkürzt wird, wenn der Abstand zwischen den distalen Enden der Schenkel zunimmt.

31. Pinzette gemäß Anspruch 30, wobei der Gelenkmechanismus eine elliptische exzentrische Achse (31) umfasst, die in der Verlängerung des zweiten Schenkels liegt, so dass eine Translationsbewegung die Länge des zweiten Schenkels verlängert, wenn sich die distalen Enden des ersten und des zweiten Schenkels in einer geschlossenen Stellung befinden.

32. Pinzette gemäß Anspruch 30, wobei der Gelenkmechanismus aus einem Doppelfedersystem besteht, wobei eine erste Feder (32) mittig zwischen dem ersten Schenkel und dem zweiten Schenkel fixiert ist und die Schenkel in einer geöffneten Stellung unter eine Vorspannung setzt und eine zweite Feder (33) am proximalen Ende der Pinzette fixiert ist, wobei die zweite Feder am proximalen Ende des zweiten Schenkels fixiert ist, wobei die zweite Feder den zweiten Schenkel hinausziehen kann, wenn sich der erste und der zweite Schenkel in einer geschlossenen Stellung befinden.

33. Pinzette gemäß einem der vorstehenden Ansprüche, wobei die Fassung einen induzierbaren Greifmechanismus umfasst, der durch darauf ausgeübten Druck betätigt wird, wobei bei Kontakt mit dem Gewebe auf die Fassung ausgeübter Druck ein leichtes Eindringen der Nadel erlaubt und die Wegnahme des Drucks einen verstärkten Zugriff auf die Nadel verursacht.

34. Pinzette gemäß einem der Ansprüche 1 bis 33, wobei die Fassung oder der Halter durch eine zur einmaligen Verwendung vorgesehene Verbindung, die bei ihrer Lösung zerstört wird, mit der Pinzette verbunden ist.

35. Chirurgisches System zur Verwendung zum Nähen von Gewebe, umfassend eine Pinzette gemäß einem der Ansprüche 1 bis 34 in Kombination mit einer chirurgischen Nadel und Faden.

36. Chirurgisches System gemäß Anspruch 35, wobei die chirurgische Nadel und der Faden als atraumatische Naht miteinander kombiniert sind.

## Revendications

1. Pince (1) destinée à être utilisée pour suturer du tissu (11), comprenant un premier bras (2) et un second bras (3) qui sont raccordés au niveau d'une extrémité proximale (4) et sont mobiles vers et à distance l'un de l'autre, et qui définissent un espace entre lesdits bras qui peut être réduit ou augmenté pour saisir le tissu, ladite pince comprenant en outre une balle (6) placée au niveau d'une extrémité distale (5) dudit bras et/ou dudit second bras, au niveau d'un côté interne et/ou inférieur de l'extrémité dudit bras, pour recevoir et fixer une aiguille chirurgicale (9) pour saisir le tissu.

2. Pince selon la revendication 1, dans laquelle ladite pince comprend en outre un raccordement à ressort (25), ledit raccordement à ressort sollicitant ledit premier bras et ledit second bras de ladite pince dans une position ouverte.

3. Pince selon l'une quelconque des revendications précédentes, dans laquelle ladite balle est appropriée pour être percée avec ladite aiguille chirurgicale.

4. Pince selon l'une quelconque des revendications précédentes, dans laquelle ladite balle est composée de matériau élastomère.

5. Pince selon la revendication 4, dans laquelle ledit matériau élastomère est du caoutchouc synthétique.

6. Pince selon l'une quelconque des revendications précédentes, dans laquelle ladite balle a un noyau creux entouré par une couche perforable (18) pour fixer ladite aiguille chirurgicale par un verrouillage de forme, moyennant quoi le matériau perforé exerce une force sur l'aiguille insérée suite à sa déformation.

7. Pince selon la revendication 6, dans laquelle ledit espace ouvert est composé d'une matière de remplissage, ladite matière de remplissage étant sélectionnée à partir des matériaux comprenant les gels, les mousses, les billes de métal ou les liquides.

8. Pince selon l'une quelconque des revendications précédentes, dans laquelle ladite balle comprend un treillis métallique (15).

9. Pince selon l'une quelconque des revendications précédentes, dans laquelle ladite balle comprend un matériau magnétique.

10. Pince selon l'une quelconque des revendications précédentes, dans laquelle ladite balle comprend un matériau adhésif.

11. Pince selon l'une quelconque des revendications précédentes, dans laquelle ladite balle comprend un dispositif de serrage.

12. Pince selon la revendication 1, dans laquelle ladite balle comprend un mécanisme de préhension inductible comprenant un mécanisme de piston plongeur dans laquelle ledit mécanisme de piston plongeur est activé lorsque lesdites extrémités distales dudit premier bras et dudit second bras s'éloignent vers une position ouverte et où lesdites extrémités distales desdits bras sont à proximité immédiate, ledit mécanisme de piston plongeur est rétracté, libérant ainsi ladite aiguille.

13. Pince selon la revendication 11, dans laquelle ledit mécanisme de piston plongeur est manuellement activé indépendamment desdites positions ouverte ou fermée de ladite pince.

14. Pince selon la revendication 1, dans laquelle ladite balle est composée d'une structure creuse composée d'au moins deux volets (21) séparés par une fente étroite (22) dans laquelle ladite aiguille chirurgicale peut être guidée par lesdits volets qui sont appropriés pour fixer ladite aiguille chirurgicale sur ladite balle et où ladite aiguille chirurgicale est libérée de ladite balle en tirant ladite aiguille chirurgicale.

15. Pince selon l'une quelconque des revendications précédentes, dans laquelle ladite balle est composée d'un matériau qui correspond au changement des conditions chimiques ou physiques telles que la température, moyennant quoi ladite balle fixe et libère ladite aiguille chirurgicale suite à un changement des conditions chimiques ou physiques sur le site de ladite balle.

16. Pince selon l'une quelconque des revendications précédentes, dans laquelle ladite balle est placée sur au moins un bras.

17. Pince selon l'une quelconque des revendications précédentes, dans laquelle ladite balle est prévue sur un support (7) qui est amovible dudit bras.

18. Pince selon l'une quelconque des revendications précédentes, dans laquelle ladite balle est composée d'un matériau biodégradable.

19. Pince selon l'une quelconque des revendications précédentes, dans laquelle lesdits premier et second bras comprennent une matière plastique et ladite pince est jetable.

20. Pince selon la revendication 19, dans laquelle lesdits premier et second bras sont assemblés par une articulation active (37).

21. Pince selon l'une quelconque des revendications précédentes, dans laquelle ladite pince est prévue avec une fixation (38) pour le raccordement amovible à un instrument médical, en particulier un bras d'une pince médicale.

22. Pince selon l'une quelconque des revendications précédentes, comprenant en outre un mécanisme pour éloigner le second bras du passage de suture.

23. Pince selon la revendication 22, dans laquelle ladite pince comprend en outre un mécanisme d'articulation approprié pour ajuster la longueur d'au moins un bras de ladite pince le long d'un axe de ladite pince avec ledit mécanisme d'articulation fixé au niveau de ladite extrémité proximale et/ou distale d'au moins un bras de ladite pince, moyennant quoi la différence de longueur le long d'un axe de ladite pince entre ledit premier bras et ledit second bras seul ou en combinaison avec ledit mécanisme d'articulation, augmente lorsque la distance entre lesdites extrémités distales desdits bras augmente.

24. Pince selon la revendication 23, dans laquelle ledit mécanisme d'articulation est composé d'une articulation (24) fixée sur un levier (23).

25. Pince selon la revendication 23 ou la revendication 24, dans laquelle ledit second bras est plus court en longueur que ledit premier bras.

26. Pince selon l'une quelconque des revendications 23 à 25, dans laquelle la différence de longueur le long dudit axe de ladite pince entre ledit premier bras et ledit second bras seul ou en combinaison avec ledit mécanisme d'articulation diminue lorsque la distance entre lesdites extrémités distales desdits bras diminue.

27. Pince selon la revendication 24, dans laquelle ledit mécanisme d'articulation est fixé au niveau de l'extrémité distale dudit premier bras de sorte que l'extrémité proximale dudit levier est fixée sur ladite articulation, dans laquelle une balle est placée au niveau d'une extrémité distale dudit levier.

28. Pince selon la revendication 24, dans laquelle ledit mécanisme d'articulation est fixé au niveau de ladite extrémité distale dudit second bras de sorte que ladite articulation est fixée au niveau d'une région médiane dans ledit levier, dans laquelle ladite extrémité proximale dudit levier est disposée de manière coulissante le long de l'intérieur dudit premier bras.

29. Pince selon la revendication 24, dans laquelle ladite articulation est fixée de manière médiane au niveau dudit premier bras et fixée de manière médiane au niveau dudit levier, dans laquelle ledit levier est parallèle et disposé de manière coulissante le long dudit second bras.

30. Pince selon la revendication 23, dans laquelle ledit mécanisme d'articulation est positionné au niveau de ladite extrémité proximale dudit instrument, moyennant quoi ledit second bras est raccourci lorsque la distance entre lesdites extrémités distales dudit bras augmente.

31. Pince selon la revendication 30, dans laquelle ledit mécanisme d'articulation comprend un axe excentrique elliptique (31) qui est continu avec ledit second bras de sorte qu'un mouvement de translation étend la longueur dudit second bras lorsque lesdites extrémités distales desdits premier et second bras sont dans une position fermée.

32. Pince selon la revendication 30, dans laquelle ledit mécanisme d'articulation est composé d'un système à deux ressorts, dans lequel un premier ressort (32) est fixé de manière médiane entre ledit premier bras et ledit second bras sollicitant lesdits bras dans une position ouverte, et un second ressort (33) fixé au niveau de l'extrémité proximale de ladite pince, dans laquelle ledit second ressort est fixé sur une extrémité proximale dudit second bras, ledit second ressort pouvant prolonger ledit second bras lorsque ledit premier et ledit second bras sont dans une position fermée.

33. Pince selon l'une quelconque des revendications précédentes, dans laquelle ladite balle comprend un mécanisme de préhension inductible activée par pression sur celui-ci, moyennant quoi la pression exercée sur la balle suite au contact avec le tissu, permet une entrée facilitée de l'aiguille et le retrait de ladite pression, provoque une préhension augmentée sur l'aiguille.

34. Pince selon l'une quelconque des revendications 1 à 33, dans laquelle ladite balle ou ledit support est raccordé à la pince par un raccordement à usage unique qui est détruit suite à sa déconnexion.

35. Système chirurgical destiné à être utilisé pour suturer du tissu, comprenant une pince selon l'une quelconque des revendications 1 à 34, en combinaison avec une aiguille chirurgicale et du fil de suture.

36. Système chirurgical selon la revendication 35, dans lequel l'aiguille chirurgicale et le fil de suture sont combinés sous la forme d'un fil de suture atraumatique.
